# EUROPEAN PATENT APPLICATION

(11) **EP 2 267 151 A1**
(43) Date of publication of application: **29.12.2010**
(21) Application number: 09727611.7
(22) Date of filing: 31.03.2009
(51) Int. Cl.: C12Q 1/06, C12Q 1/68, G01N 33/569

(54) **VIRUS TITRATION METHOD**

(30) Priority: 31.03.2008 JP 2008093288
(71) Applicant: Japan Tobacco, Inc., Tokyo 105-8422 (JP)
(72) Inventor: TAKAKURA, Yoshimitsu, Iwata-shi Shizuoka 438-0802 (JP); ICHIKAWA, Masako, Iwata-shi Shizuoka 438-0802 (JP); KONDO, Kazuhiro, Tokyo 105-8461 (JP); SHIMIZU, Akihiro, Tokyo 152-0032 (JP)
(74) Representative: Reinhard - Skuhra - Weise & Partner GbR
(86) International application number: PCT/JP2009/057039
(87) International publication number: WO 2009/123347

(57) **Abstract**

The present invention relates to a method of quantitatively determining the number of human herpesvirus (HHV) collected from a body fluid and a kit for performing the method. Conventionally, a trained technician has been required to accurately quantitatively determine a number of HHV collected from a body fluid. The method of the present invention is a novel method of quantitative determination that enables measurement of a number of HHV in a body fluid to be simply, accurately, and efficiently determined. The method of the present invention can enable continuous evaluation of the number of HHV in body fluids and, therefore, can be applied to quantitative evaluation of the accumulation of fatigue.

SEQUENCE LISTING

<110> Japan Tobacco Inc.

<120> Quantitation method of virus

<130> P30423-WOEP

<140> 09727611.7
<141> 2009-03-31

<150> JP 2008-93288
<151> 2008-03-31

<160> 16

<170> PatentIn version 3.1

<210> 1
<211> 23
<212> DNA
<213> Artificial

<220>
<223> primer for amplifying HHV-6 U65/66 region

<400> 1
gacaatcaca tgcctggata atg 23

<210> 2
<211> 24
<212> DNA
<213> Artificial

<220>
<223> primer for amplifying HHV-6 U65/66 region

<400> 2
tgtaagcgtg tggtaatgga ctaa 24

<210> 3
<211> 24
<212> DNA
<213> Artificial

<220>
<223> Taqman probe for quantitation of HHV-6 U65/66 region: FAM is link
ed to 5'-end, and TAMRA is linked to 3'-end

<400> 3
agcagctggc gaaaagtgct gtgc 24

<210> 4
<211> 17
<212> DNA
<213> Artificial

<220>
<223> primer for amplifying EGFP gene

<400> 4
ctgctgcccg acaacca 17

<210> 5
<211> 19

<212>  DNA
<213>  Artificial

<220>
<223>  primer for amplifying EGFP gene

<400>  5
tgtgatcgcg cttctcgtt                                                    19


<210>  6
<211>  21
<212>  DNA
<213>  Artificial

<220>
<223>  Taqman probe for quantitation of EGFP gene: FAM is linked to 5'-e
       nd, and TAMRA is linked to 3'-end

<400>  6
ctgagcaccc agtccgccct g                                                 21


<210>  7
<211>  24
<212>  DNA
<213>  Artificial

<220>
<223>  primer for amplifying HHV-6 U5 region

<400>  7
cgaagaaaag tagcacaggt ctcc                                              24


<210>  8
<211>  24
<212>  DNA
<213>  Artificial

<220>
<223>  primer for amplifying HHV-6 U5 region

<400>  8
accgtgtcat aaatgctgag ttgg                                              24


<210>  9
<211>  21
<212>  DNA
<213>  Artificial

<220>
<223>  Taqman probe for quantitation of HHV-6 U5 region; FAM is linked t
       o 5'-end, and TAMRA is linked to 3'-end

<400>  9
aggcacccgt tccgccccag c                                                 21


<210>  10
<211>  432
<212>  DNA
<213>  Pleurotus cornucopiae

<220>
<221>  CDS
<222>  (1)..(432)
<223>  Tamavidin 1

<400>  10
atg aaa gac gtc caa tct ctc ctc acc gga acc tgg tac aat gaa ctc        48
Met Lys Asp Val Gln Ser Leu Leu Thr Gly Thr Trp Tyr Asn Glu Leu
1               5                   10                  15

ggc tca aca atg aat ttg act gca aat aaa gac ggt tcg ctc acc gga        96
Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct       144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga       192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca       240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc       288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc       336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag       384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga       432
Lys Ile Ala Gln Ala Gln Leu Leu Arg Arg Gly Leu Lys Phe Glu
    130                 135                 140


<210>  11
<211>  143
<212>  PRT
<213>  Pleurotus cornucopiae

<400>  11

Met Lys Asp Val Gln Ser Leu Leu Thr Gly Thr Trp Tyr Asn Glu Leu
1               5                   10                  15


Gly Ser Thr Met Asn Leu Thr Ala Asn Lys Asp Gly Ser Leu Thr Gly
            20                  25                  30


Thr Tyr His Ser Asn Val Gly Glu Val Pro Pro Thr Tyr His Leu Ser
        35                  40                  45


Gly Arg Tyr Asn Leu Gln Pro Pro Ser Gly Gln Gly Val Thr Leu Gly
    50                  55                  60


Trp Ala Val Ser Phe Glu Asn Thr Ser Ala Asn Val His Ser Val Ser
65                  70                  75                  80


Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu
    130                 135                 140

<210>  12
<211>  426
<212>  DNA
<213>  Pleurotus cornucopiae

<220>
<221>  CDS
<222>  (1)..(426)
<223>  Tamavidin 2

<400>  12
atg tca gac gtt caa tct tca ctc acc gga acc tgg tac aat gaa ctc      48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga      96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct      144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg      192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg      240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg      288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt      336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc      384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa              426
Ala His Ala Gln Leu His Cys Arg Ala Pro Arg Leu Lys
    130                 135                 140

<210>  13
<211>  141
<212>  PRT
<213>  Pleurotus cornucopiae

<400>  13

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

<210>  14
<211>  22
<212>  DNA
<213>  Artificial

<220>
<223>  primer for amplifying HHV-7 U37 region

<400>  14
cggaagtcac tggagtaatg ac                                                22

<210>  15
<211>  20
<212>  DNA
<213>  Artificial

<220>
<223>  primer for amplifying HHV-7 U37 region

<400>  15
ccaatccttc cgaaaccgat                                                   20

<210>  16
<211>  25
<212>  DNA
<213>  Artificial

<220>
<223>  Taqman probe for quantitation of HHV-7 U37 region; FAM is linked
       to 5'-end, and TAMRA is linked to 3'-end

<400>  16
cctcgcagat tgcttgttgg ccatg                                              25

SEQUENCE LISTING

<110> Japan Tobacco Inc.
Virus Ikagaku Kenkyusho Inc.

<120> Quantitation method of virus

<130> FA0392-09035

<150> JP 2008-93288
<151> 2008-03-31

<160> 16

<170> PatentIn version 3.1

<210> 1
<211> 23
<212> DNA
<213> Artificial

<220>
<223> primer for amplifying HHV-6 U65/66 region

<400> 1
gacaatcaca tgcctggata atg 23

<210> 2
<211> 24
<212> DNA
<213> Artificial

<220>
<223> primer for amplifying HHV-6 U65/66 region

<400> 2
tgtaagcgtg tggtaatgga ctaa 24

<210> 3
<211> 24
<212> DNA
<213> Artificial

<220>
<223> Taqman probe for quantitation of HHV-6 U65/66 region: FAM is link
ed to 5'-end, and TAMRA is linked to 3'-end

<400> 3
agcagctggc gaaaagtgct gtgc 24

<210> 4
<211> 17
<212> DNA
<213> Artificial

<220>
<223> primer for amplifying EGFP gene

<400> 4
ctgctgcccg acaacca 17

<210> 5
<211> 19
<212> DNA
<213> Artificial

<220>
<223> primer for amplifying EGFP gene

<400> 5
tgtgatcgcg cttctcgtt 19

<210> 6
<211> 21
<212> DNA
<213> Artificial

<220>
<223> Taqman probe for quantitation of EGFP gene: FAM is linked to 5'-e
nd, and TAMRA is linked to 3'-end

<400> 6
ctgagcaccc agtccgccct g 21

<210> 7
<211> 24
<212> DNA
<213> Artificial

<220>
<223> primer for amplifying HHV-6 U5 region

<400> 7
cgaagaaaag tagcacaggt ctcc 24

<210> 8
<211> 24
<212> DNA
<213> Artificial

<220>

<223>  primer for amplifying HHV-6 U5 region

<400>  8
accgtgtcat aaatgctgag ttgg                                              24

<210>  9
<211>  21
<212>  DNA
<213>  Artificial

<220>
<223>  Taqman probe for quantitation of HHV-6 U5 region; FAM is linked t
       o 5'-end, and TAMRA is linked to 3'-end

<400>  9
aggcacccgt tccgccccag c                                                 21

<210>  10
<211>  432
<212>  DNA
<213>  Pleurotus cornucopiae

<220>
<221>  CDS
<222>  (1)..(432)
<223>  Tamavidin 1

<400>  10
atg aaa gac gtc caa tct ctc ctc acc gga acc tgg tac aat gaa ctc        48
Met Lys Asp Val Gln Ser Leu Leu Thr Gly Thr Trp Tyr Asn Glu Leu
1               5                   10                  15

ggc tca aca atg aat ttg act gca aat aaa gac ggt tcg ctc acc gga        96

Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct 144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga 192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca 240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc 288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc 336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag 384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga 432
Lys Ile Ala Gln Ala Gln Leu Leu Arg Arg Gly Leu Lys Phe Glu
    130                 135                 140

<210> 11
<211> 143
<212> PRT
<213> Pleurotus cornucopiae

<400> 11

Met Lys Asp Val Gln Ser Leu Leu Thr Gly Thr Trp Tyr Asn Glu Leu
1               5                   10                  15

Gly Ser Thr Met Asn Leu Thr Ala Asn Lys Asp Gly Ser Leu Thr Gly
            20                  25                  30

Thr Tyr His Ser Asn Val Gly Glu Val Pro Pro Thr Tyr His Leu Ser
        35                  40                  45

Gly Arg Tyr Asn Leu Gln Pro Pro Ser Gly Gln Gly Val Thr Leu Gly
    50                  55                  60

Trp Ala Val Ser Phe Glu Asn Thr Ser Ala Asn Val His Ser Val Ser
65                  70                  75                  80

Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu

    130                 135                 140

<210> 12
<211> 426
<212> DNA
<213> Pleurotus cornucopiae

<220>
<221> CDS
<222> (1)..(426)
<223> Tamavidin 2

<400> 12
atg tca gac gtt caa tct tca ctc acc gga acc tgg tac aat gaa ctc 48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga 96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct 144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg 192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg 240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg 288

Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt 336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc 384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa 426
Ala His Ala Gln Leu His Cys Arg Ala Pro Arg Leu Lys
    130                 135                 140

<210> 13
<211> 141
<212> PRT
<213> Pleurotus cornucopiae

<400> 13

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly

    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

<210> 14
<211> 22
<212> DNA
<213> Artificial

<220>
<223> primer for amplifying HHV-7 U37 region

<400> 14
cggaagtcac tggagtaatg ac 22

<210> 15

<211>  20
<212>  DNA
<213>  Artificial

<220>
<223>  primer for amplifying HHV-7 U37 region

<400>  15
ccaatccttc cgaaaccgat                                                    20


<210>  16
<211>  25
<212>  DNA
<213>  Artificial

<220>
<223>  Taqman probe for quantitation of HHV-7 U37 region; FAM is linked
       to 5'-end, and TAMRA is linked to 3'-end

<400>  16
cctcgcagat tgcttgttgg ccatg                                              25

## Description

### TECHNICAL FIELD

The present invention relates to a method of quantitatively determining the number of human herpesvirus (HHV) collected from a body fluid and a kit for performing the method.

### BACKGROUND ART

Accumulation of "fatigue" causes many serious problems such as death from overwork, suicide, and lifestyle-related diseases. However, scientific and medical studies on "fatigue" are significantly retarded as compared to medical studies in other fields, and not even a partial solution has been provided for problems caused by "fatigue" in the health of individuals and society as a whole.

A main reason for the delay in studies on "fatigue" and development of prevention and treatment thereof is that a method for objectively measuring "fatigue" has not been proposed. In particular, a method for measuring "accumulation of fatigue" or "medium- and long-term continuous fatigue," which most severely affects life and health, has not been established, and there is a high demand for such a method.

One of the inventors of the present application has developed a method for measuring accumulation of fatigue by determining the amount of a human herpesvirus, which is reactivated, and released into saliva (WO2006/006634). Previous studies have revealed that fatigue accumulated by various types of stresses for a term of one week or more can be quantitatively determined by this method. Furthermore, in this method, saliva is preferably used as a sample. Regarding saliva, it is known that a content of endogenous biotin in the salivary gland is high (Green, M., et al., J. Clin. Pathol., (1992) 45(9): 788-790).

Conventional methods for quantitatively determining viruses are, for example, determination of an amount of viral DNA by PCR (Kido, S., et al., J. Med. Virol., (1990) 32: 139-142) or by double-nested PCR (Kondo, K., et al., J. Infect. Dis., (1993) 167: 1197-1200). In addition, there is disclosed a method of quantitative determination of a retrovirus, which utilizes a retrovirus having viral nucleic acid containing a marker sequence as an internal standard when the amount of the viral DNA is measured by quantitative PCR (WO95/034684).

Furthermore, a known method for measuring an amount of viral protein involves, for example, immunoassay using an antibody against the viral protein. A typical example of the immunoassay is sandwich ELISA (Gerna, G., et al., J. Clin. Microbiol., (1983) 17: 942-944).

Meanwhile, a known method for raising a concentration of a virus in a solution and thereby concentrating the virus is, for example, ultracentrifugation. Unfortunately, this method requires use of expensive equipment and a long time for separation and thus requires a large amount of work for carrying out the method. In addition, a method of concentrating a virus is known where the virus is precipitated by ammonium sulfate or polyethylene glycol. Unfortunately, such a method has a disadvantage in that since the reagent used in the method inhibits PCR subsequently performed for detecting the virus, purification of the sample is necessary after precipitation of the virus. Another method disclosed for concentrating a virus is, for example, use of magnetic particles to which a mannose-binding lectin is immobilized (Japanese Patent Public Disclosure No. 2002-165591). In addition, disclosed is a method using streptavidin and a biotinylated lectin that enhances the retroviral titer or isolates a retrovirus from a sample (WO2001/079456).

### PRIOR ART REFERENCES

Patent Document 1: International Publication No. WO2006/006634
Patent Document 2: International Publication No. WO95/034684
Patent Document 3: Japanese Patent Public Disclosure No. 2002-165591
Patent Document 4: International Publication No. WO2001/079456
Non-patent Document 1: Green, M., et al., J. Clin. Pathol., (1992) 45(9): 788-790
Non-patent Document 2: Kido, S., et al., J. Med. Virol., (1990) 32: 139-142
Non-patent Document 3: Kondo, K., et al., J. Infect. Dis., (1993) 167: 1197-1200
Non-patent Document 4: Gerna, G., et al., J. Clin. Microbiol., (1983) 17: 942-944

### DISCLOSURE OF THE INVENTION

### PROBLEMS TO BE SOLVED BY THE INVENTION

The inventors have measured amounts of human herpesviruses (hereinafter, abbreviated as HHVs) in body fluids by a known method in order to quantitatively evaluate degrees of fatigue in daily life. In this regard, the inventors have found that the numbers of the HHVs in the samples were very low in some cases, that is, the number of human herpesvirus 6 (HHV-6) was less than 100 copies/mL and the number of human herpesvirus 7 (HHV-7) was also less than 100 copies/mL, and that viral DNAs were lost halfway through measurement in many cases and thereby technical training was necessary to ensure accurate and efficient quantitative determination. It was therefore difficult to determine quantitatively a large number of samples readily by preparing a kit for a known method of quantitatively determining an HHV. Accordingly, it is an object of the present invention to solve the above-mentioned problems in virus quantitative determination.

### MEANS FOR SOLVING THE PROBLEM

The inventors have conducted intensive studies and, as a result, they have found that a sample, which comprises a virus in a concentration that can readily be measured by a method such as PCR, LAMP, or ELISA, can be prepared by mixing a body fluid with a carrier immobilized with a substance capable of binding to an HHV, thereby making the target virus bind to the substance, and collecting the carrier by means of magnetic force or centrifugal treatment.

Furthermore, the inventors have succeeded in providing accurate quantitative determination by adding a certain amount of virus serving as a standard to a sample in the course of the quantitative determination and conducting a series of steps. This method ensures simple and further accurate quantitative determination by using a mutated HHV as the standard virus.

Based on the above-mentioned findings, the present invention provides a novel method of quantitative determination that can measure an HHV in a body fluid simply and accurately, and provides a kit for performing the method of the present invention. That is, the present invention relates to the following aspects.

Embodiment 1: A method of quantitatively determining a number of human herpesvirus collected from a body fluid, the method including the steps of:
(1) adding a standard virus to the collected body fluid, the concentration of the standard virus being determined in advance;
(2) bringing the solution prepared in step (1) into contact with a virus-binding substance, wherein (i) the virus-binding substance is linked to a molecule capable of binding to a carrier and is immobilized to the carrier through the molecule and thereby makes the virus particles bound to the virus-binding substance bind indirectly to the carrier, or (ii) the virus-binding substance is directly immobilized to a carrier and thereby makes the virus particles bound to the virus-binding substance bind to the carrier;
(3) separating the carrier from the solution prepared in step (1);
(4) quantitatively determining the number of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

Embodiment 2: A method of quantitatively determining the number of human herpesvirus collected from a body fluid, the method including the steps of:
(1) adding a standard virus to the collected body fluid, the concentration of the standard virus being determined in advance;
(2) bringing the solution prepared in step (1) into contact with a lectin, wherein (i) the lectin is linked to a molecule capable of binding to a carrier and is immobilized to the carrier through the molecule and thereby makes the virus particles bound to the lectin bind indirectly to the carrier, or (ii) the lectin is directly immobilized to a carrier and thereby makes the virus particles bound to the lectin bind to the carrier;
(3) separating the carrier from the solution prepared in step (1);
(4) quantitatively determining the numbers of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

Embodiment 3: A method of quantitatively determining the number of human herpesvirus collected from saliva, the method including the steps of:
(1) adding a standard virus to the collected saliva, the concentration of the standard virus being determined in advance;
(2) mixing the solution prepared in step (1) with a biotinylated lectin for bringing virus particles into contact with the biotinylated lectin, and then adding biotin-binding protein-immobilized beads thereto to make the virus particles bound to the biotinylated lectin bind to the beads;
(3) separating the beads from the solution prepared in step (1);
(4) quantitatively determining the numbers of virus recovered from the separated beads; and
(5) evaluating the recovery rate by comparing the number of the collected standard virus with the number of the standard virus added in step (1), and determining the number of the human herpesvirus collected from the saliva based on the number of the human herpesvirus determined in step (4) and the recovery rate.

Embodiment 4: The method according to any one of embodiments 1 to 3, wherein the human herpesvirus is human herpesvirus 6 (HHV-6) or human herpesvirus 7 (HHV-7).

Embodiment 5: The method according to any one of embodiments 1 to 3, wherein the standard virus is a recombinant virus derived from HHV-6 or HHV-7.

Embodiment 6: The method according to embodiments 2 or 3, wherein the lectin is a lectin that binds to a sugar chain containing at least one ofN-acetylgalactosamine (GalNAc), α2,6-linked sialic acid (Siaα2,6), and N-acetylglucosamine (GlcNAc).

Embodiment 7: The method according to embodiment 6, wherein the lectin is selected from the group consisting of SBA (derived from soybean), SSA (derived from *Sambucus sieboldiana*), DSA (derived from *Datura stramonium*), and WGA (derived from wheat germ).

Embodiment 8: The method according to any one of embodiments 1 to 3, wherein the quantitative determination of the number of virus in step (4) is performed by a procedure selected from the group consisting of PCR, LAMP, and ELISA.

Embodiment 9: A method of quantitatively determining the number of human herpesvirus 6 (HHV-6) or human herpesvirus 7 (HHV-7) collected from saliva, the method including the steps of:
(1) adding a standard virus having a predetermined concentration to the collected saliva, such that the concentration of the standard virus is 10 to 100000 genome copies/mL, wherein the standard virus is a recombinant virus derived from HHV-6 or HHV-7;
(2) mixing the solution prepared in step (1) with a biotinylated lectin for bringing virus particles into contact with the biotinylated lectin, and then adding biotin-binding protein-immobilized beads thereto to make the virus particles bound to the biotinylated lectin bind to the beads, wherein the lectin is selected from the group consisting of SBA (derived from soybean), SSA (derived from *Sambucus sieboldiana*), DSA (derived from *Datura stramonium*), and WGA (derived from wheat germ);
(3) separating the beads from the solution prepared in step (1);
(4) quantitatively determining the number of virus recovered from the separated beads by a procedure selected from the group consisting of PCR, LAMP, and ELISA; and
(5) evaluating the recovery rate by comparing the number of the collected standard virus with the number of the standard virus added in step (1), and determining the number of the human herpesvirus collected from the saliva based on the number of the human herpesvirus determined in step (4) and the recovery rate.

Embodiment 10: A method of quantitatively determining the number of human herpesvirus collected from a body fluid, the method including the steps of:
(1) adding a standard virus to the collected body fluid, the concentration of the standard virus being determined in advance;
(2) bringing the solution prepared in step (1) into contact with a virus-binding substance that is directly immobilized to nanobeads having a diameter of 10 to 100 nm to make virus particles bind to the nanobeads;
(3) separating the nanobeads from the solution prepared in step (1);
(4) quantitatively determining the number of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

Embodiment 11: A kit for quantitatively determining the number of human herpesvirus collected from a body fluid, the kit including:
a biotinylated lectin; and
biotin-binding protein-immobilized beads.

Embodiment 12: A kit for quantitatively determining the number of human herpesvirus collected from a body fluid, the kit including:
a biotinylated lectin;
biotin-binding protein-immobilized beads; and
a standard virus, the concentration of the standard virus being determined in advance.

Embodiment 13: The kit according to embodiment 11 or 12, wherein the lectin is a lectin that binds to a sugar chain containing at least one ofN-acetylgalactosamine (GalNAc), α2,6-linked sialic acid (Siaα2,6), and N-acetylglucosamine (GlcNAc).

Embodiment 14: The kit according to embodiment 13, wherein the lectin is selected from the group consisting of SBA (derived from soybean), SSA (derived from *Sambucus sieboldiana*), DSA (derived from *Datura stramonium*), and WGA (derived from wheat germ); and the standard virus is a recombinant virus derived from HHV-6 or HHV-7.

Embodiment 15: A method of quantitatively determining the number of human herpesvirus collected from a body fluid, the method including the steps of:
(1) bringing the collected body fluid into contact with a virus-binding substance, wherein (i) the virus-binding substance is linked to a molecule capable of binding to a carrier and is immobilized to the carrier through the molecule and thereby makes the virus particles bound to the virus-binding substance bind indirectly to the carrier, or (ii) the virus-binding substance is directly immobilized to a carrier and thereby makes the virus particles bound to the virus-binding substance bind to the carrier;
(2) separating the carrier from the solution prepared in step (1); and
(3) quantitatively determining the amount of the virus recovered from the separated carrier.

Embodiment 16: A method of quantitatively determining the number of human herpesvirus collected from saliva, the method including the steps of:
(1) mixing the collected saliva with a biotinylated lectin for bringing a virus into contact with the biotinylated lectin, and then adding biotin-binding protein-immobilized beads thereto to make the virus bound to the biotinylated lectin bind to the beads;
(2) separating the beads from the solution prepared in step (1); and
(3) quantitatively determining the number of the virus recovered from the separated beads.

### ADVANTAGES OF THE INVENTION

According to the present invention, an HHV, which cannot be readily quantitatively determined because of a very low concentration of the HHV in a body fluid and therefore requires a high degree of training and skill, can be quantitatively determined simply and more accurately.

### BRIEF DESCRIPTION OF THE DRAWINGS

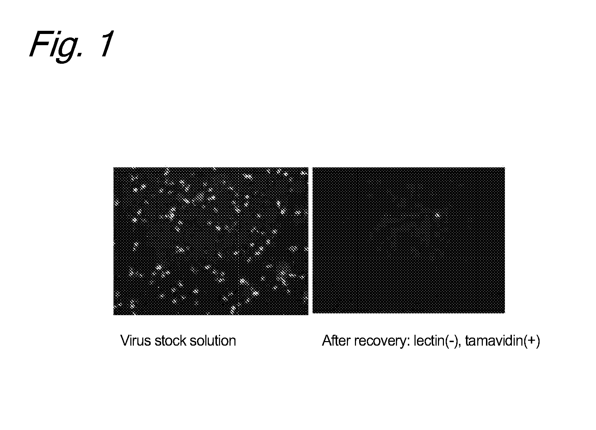

Fig. 1 is fluorescence photomicrographs showing the results of a test for non-specific adsorption of HHV-6 to tamavidin beads. The bright green spots show MT-4 cells serving as indicator cells infected with EGFP recombinant HHV-6. One spot represents one infectious virus particle (the difference in brightness of each spot depends on a difference in gene expression of the virus after infection). The left photograph shows the results after a HHV-6 virus solution (stock solution) was applied to MT-4 cells. The right photograph shows the results after a HHV-6 virus solution (stock solution) was brought into contact with tamavidin beads in the absence of a lectin, and then the virus adsorbed to the tamavidin beads was applied to MT-4 cells.

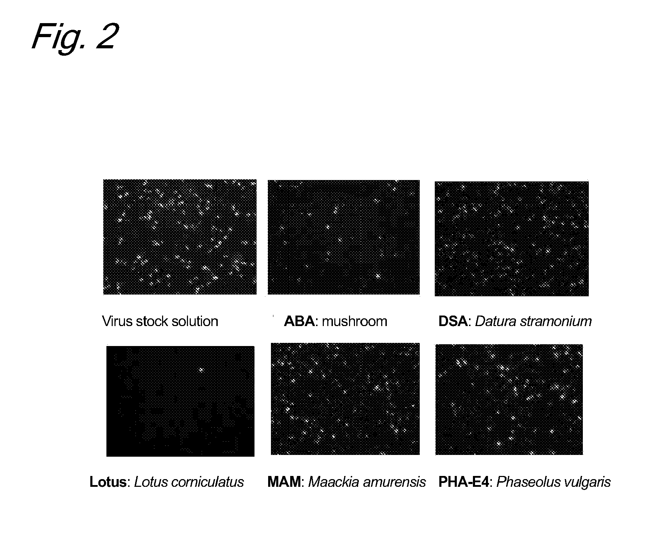

Fig. 2 is fluorescence photomicrographs showing the results of a test for effects of each of lectins, i.e., ABA, DSA, Lotus, MAM, or PHA-E4 on concentration of HHV-6. The bright green spots show MT-4 cells serving as indicator cells infected with EGFP recombinant HHV-6. One spot represents one infectious virus particle (the difference in brightness of each spot depends on a difference in gene expression of the virus after infection).

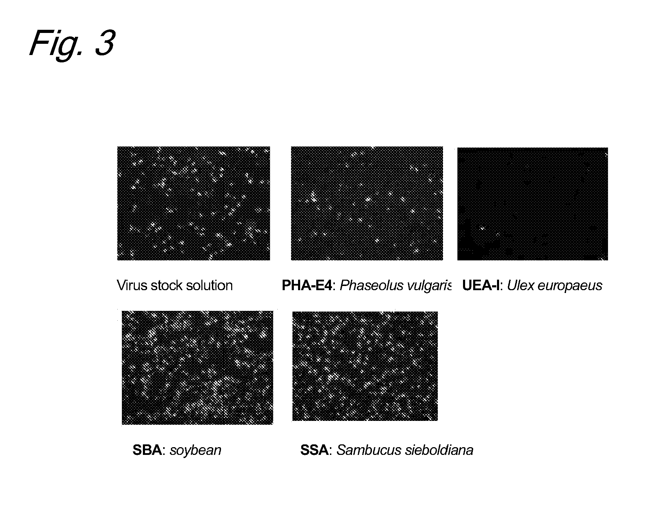

Fig. 3 is fluorescence photomicrographs showing the results of a test for effects of each of lectins, i.e., PHA-L4, UEA-I, SBA, or SSA on concentration of HHV-6. The bright green spots show MT-4 cells serving as indicator cells infected with EGFP recombinant HHV-6. One spot represents one infectious virus particle (the difference in brightness of each spot depends on a difference in gene expression of the virus after infection).

### EMBODIMENTS OF THE INVENTION

The present invention will be described in further detail below.

### 1. Method of quantitative determination of HHV in body fluid

The present invention provides a method of quantitatively determining a number of human herpesvirus collected from a body fluid, the method including the steps of:
(1) bringing the collected body fluid into contact with a virus-binding substance, wherein (i) the virus-binding substance is linked to a molecule capable of binding to a carrier and is immobilized to the carrier through the molecule and thereby makes the virus particles bound to the virus-binding substance bind indirectly to the carrier, or (ii) the virus-binding substance is directly immobilized to a carrier and thereby makes the virus particles bound to the virus-binding substance bind to the carrier;
(2) separating the carrier from the solution prepared in step (1); and
(3) quantitatively determining the amount of the virus recovered from the separated carrier.

Furthermore, the present invention provides a method of quantitatively determining the number of human herpesvirus collected from a body fluid, the method including the steps of:
(1) adding a standard virus to the collected body fluid, the concentration of the standard virus being determined in advance;
(2) bringing the solution prepared in step (1) into contact with a virus-binding substance, wherein (i) the virus-binding substance is linked to a molecule capable of binding to a carrier and is immobilized to the carrier through the molecule and thereby makes the virus particles bound to the virus-binding substance bind indirectly to the carrier, or (ii) the virus-binding substance is directly immobilized to a carrier and thereby makes the virus particles bound to the virus-binding substance bind to the carrier;
(3) separating the carrier from the solution prepared in step (1);
(4) quantitatively determining the number of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

Each configuration of the method of the present invention will be described in detail below.

### Virus

The virus to be quantitatively determined by the method of the present invention is a virus that can quantitatively indicate human fatigue, and is specifically a virus belonging to Herpesviridae, with which human beings are infected, that is, a human herpesvirus (hereinafter, also referred to as HHV.) Preferably, the virus is one or more viruses selected from the group consisting of human herpesvirus 6 (variants A and B of human herpesvirus 6: hereinafter, also collectively referred to as HHV-6), human herpesvirus 7 (hereinafter, also referred to as HHV-7), cytomegalovirus (also called human herpesvirus 5), and Epstein-Barr virus (hereinafter, also referred to as EBV.) More preferably, the virus is HHV-6 or HHV-7, and most preferably HHV-6.

The human herpesvirus is known as a double strand DNA virus with which human beings are infected.. HHV-6 is a virus having a diameter of about 200 nm.

### Body fluid

The body fluid to be analyzed by the method of the present invention may be any body fluid collected from a living body, such as blood, saliva, serum, semen, breast milk, pharynx wiping liquid, cerebrospinal fluid, ascitic fluid, perspiration, tear, and urine. Saliva is preferred. In this description, the body fluid to be analyzed by the method of the present invention is also simply referred to as a "sample."

As the method of collecting saliva, for example, a method of collecting mucus by wiping a pharyngeal region with a swab, a method of collecting saliva directly spewed into a sampling tube, or a method using a saliva sampler, such as Salivette (Sarstedt), can be employed. The method using Salivette is preferred. In this case, saliva is collected by putting cotton into the mouth and impregnating the cotton with saliva, and then the cotton impregnated with saliva is transferred to a centrifugal tube and centrifuged to collect the saliva.

The oral cavity is treated before collecting saliva by, for example, by refraining from food intake for a long time and placing at rest, or rinsing with water immediately before collection of saliva. The method of rinsing the oral cavity with water immediately before the collection of saliva is preferred.

Body fluids other than saliva can also be collected by any method known to those skilled in the art.

The collected body fluid may be subjected to appropriate treatment, such as dilution, filtration, or centrifugation, according to need before the quantitative determination of viral particles or viral DNA as long as the treatment does not affect the determination. Alternatively, if the virus is not immediately measured, the body fluid may be refrigerated or frozen for storage according to need by a method known to those skilled in the art until being subjected to determination.

### Virus-binding substance

In the present invention, the virus-binding substance is a substance capable of binding to an HHV. Examples of a substance capable of binding to an HHV include lectins and antibodies against the HHV.

In this description, the term "lectin" refers to a saccharide-binding protein that recognizes a sugar chain and binds thereto. Each lectin selectively binds to a specific sugar chain.

The lectin used in the present invention is not limited particularly as long as it can bind to an HHV, but is preferably a lectin having a high affinity to a virus to be quantitatively determined and showing a high recovery rate of the virus. More preferably, the lectin is a lectin that binds to a sugar chain containing at least one ofN-acetylgalactosamine (GalNAc), α2,6-linked sialic acid (Siaα2,6), and N-acetylglucosamine (GlcNAc) and is most preferably a lectin selected from the group consisting of SBA (a lectin derived from soybean, binding sugar chain: sugar chain containing N-acetylgalactosamine (GalNAc)), SSA (a lectin derived from *Sambucus sieboldiana,* binding sugar chain: sugar chain containing α2,6-linked sialic acid (Siaα2,6)), DSA (a lectin derived from *Datura stramonium,* binding sugar chain: sugar chain containing N-acetylglucosamine (GlcNAc)), and WGA (a lectin derived from wheat germ, binding sugar chain: sugar chain containing N-acetylglucosamine (GlcNAc) or sialic acid). In particular, if the virus to be quantitatively determined is HHV-6, the lectin is more preferably SBA or WGA.

The antibody against an HHV used in the present invention is not particularly limited as long as it can bind to the HHV and may be either a polyclonal antibody or a monoclonal antibody.

The virus-binding substance in one embodiment of the present invention may be used by being directly immobilized to a carrier.

In another embodiment, the virus-binding substance may be used by being indirectly immobilized to a carrier. An example of indirect immobilization is binding of the virus-binding substance and the carrier through binding between biotin and a biotin-binding protein. More specifically, the indirect immobilization of the virus-binding substance to the carrier can be achieved by biotinylating the virus-binding substance and, at the same time, linking the biotin-biding protein to the surface of the carrier and then binding the biotin and the biotin-binding protein.

### Carrier

In the present invention, the carrier is not limited particularly as long as it is a solid or insoluble material (for example, a material that can be separated from a reaction mixture by filtration, precipitation, magnetic separation, or other means).

Examples of the material constituting the solid carrier include, but are not limited to, cellulose, Teflon (registered trademark), nitrocellulose, agaroses, highly cross-linked spherical agaroses, dextran, chitosan, polystyrene, polyacrylamide, polyesters, polycarbonates, polyamides, polypropylene, nylons, polydivinylidene difluoride, latex, silica, glass, glass fiber, gold, platinum, silver, copper, iron, stainless steel, ferrite, silicon wafers, polyethylene, polyethyleneimine, polylactic acid, resins, polysaccharides, proteins (e.g., albumin), carbon, and combinations thereof.

Examples of the shape of the solid carrier include, but not limited to, beads, magnetic beads, thin films, microcapillary tubes, filters, plates, microplates, carbon nanotubes, and sensor chips. A planar solid carrier, such as a thin film or a plate, may be provided with, for example, pits, grooves, filter bottoms, as known in the art.

In one embodiment of the present invention, the magnetic beads can have a spherical diameter in the range of from about 10 nm to about 1 mm. In a preferred embodiment, the magnetic beads have a diameter in the ranges of from about 25 nm to about 1 mm and from about 50 nm to about 100 µm. The size of the magnetic beads can be selected according to specific application.

In one embodiment of the present invention, beads made of a highly cross-linked spherical agaroses, such as sepharose, can have a diameter in a range of from about 24 to about 165 µm. In a preferred embodiment, the highly cross-linked spherical agarose beads have a diameter in a range of from about 24 to about 44 µm. The size of the highly cross-linked spherical agarose beads can be selected according to specific application.

Examples of the solid carrier having a hydrophobic surface include polystyrene latex beads that are commercially available from Polysciences Inc. or Spherotech Inc.

Examples of silica (SiO₂)-treated or silica (SiO₂)-based solid carrier include superparamagnetic silica beads that are available from Polysciences Inc. In addition, M-280 can be used, which is commercially available from Dynal Biotech LLC.

Examples of the magnetic beads having a hydrophilic surface include beads commercially available from Polysciences Inc. under a trade name of Biomag (registered trademark) carboxyl and beads (MC02N/2928) available from Bangs Laboratory Inc. In addition, M-270 can be used, which is commercially available from Dynal Biotech LLC.

### Direct binding of virus-binding substance and carrier

In one embodiment of the present invention, the virus-binding substance linked directly to the carrier can be used. In this case, from a viewpoint of increasing a likelihood of association between virus particles and a lectin-immobilized carrier, the carrier should be nanobeads of a size that permits Brownian motion. The preferred spherical diameter of the nanobeads ranges from 10 to 100 nm, more preferably from 30 to 70 nm. In addition, in this case, the nanobeads preferably contain a magnetic material so that the nanobeads can be collected readily with a magnet. Examples of the nanobeads include, but are not limited to, magnetic nanobeads, such as MACS MicroBeads (diameter: 50 nm) available from Miltenyi Biotech Inc.

Since the virus-binding substance is a protein in many cases, the virus-binding protein and the carrier can be linked by a coupling process between a protein and a carrier known to those skilled in the art. For example, the protein and the carrier can be linked by modifying the carrier surface so as to expose carboxyl groups at the surface and coupling the carboxyl groups to amino groups of the protein in the presence of 1-ethyl-3-(3-dimethylaminopropyl)carbodiimide (EDC), which is a cross-linking reagent. Alternatively, for example, the carboxyl groups of the carrier surface and the amino groups of the protein can be bound by actively esterifying the carboxyl groups of the carrier surface by N-hydroxysuccinimide (NHS) and mixing the carrier with the protein in a buffer that does not containing primary amino groups and having a pH of 6.5 to 9.

Alternatively, the amino groups of the carrier surface and the amino groups of the protein can be linked by using a cross-linking reagent, BS3 (bis[sulfosuccinimidyl] suberate) or DSS (disuccinimidyl suberate); or the amino groups of the carrier surface and the thiol groups of the protein can be bound using a cross-linking reagent, SPDP (N-succinimidyl 3-[2-pyridyldithio]propionate) or GMBS (N-(4-maleimidebutyryloxy)succinimide).

### Indirect binding of virus-binding substance and carrier

In another embodiment of the present invention, the virus-binding substance linked indirectly to a carrier may be used.

For example, the virus-binding substance can be linked indirectly to the carrier, by being biotinylated while a biotin-binding protein is immobilized to the carrier surface, and then by being bound to the biotin-binding protein.

In this case, use of beads as the carrier is preferable. A spherical diameter of the beads is preferably in the range of 0.1 to 100 µm, more preferably 0.5 to 10 µm, and most preferably 1 to 5 µm

In this description, the term "biotin" is a general name of D-[(+)-cis-hexahydro-2-oxo-1H-thieno-(3,4)-imidazole-4-valeric acid]. The biotin is a water-soluble vitamin classified into a Vitamin B group and is also called Vitamin B₇, or called Vitamin H or coenzyme R in some cases. The biotin binds to avidin, which is is a glycoprotein contained in albumen, with very high affinity.

In this description, the term "biotin" also includes biotin analogs such as iminobiotin (Hofmann, et al., Proc. Natl. Acad. Sci. USA, (1980) 77: 4666-4668), desthiobiotin (Hirsch, et al., Anal. Biochem., (2002) 308: 343-357), biocytin, and biotin sulfoxide, in addition to the above-mentioned biotin.

The virus-binding substance such as a lectin may be biotinylated by using a biotinylation reagent. Examples of usable biotinylation reagent include, but not limited to, Pierce products (in parentheses, the linker length and the reactive group in this order), such as EZ-Link (registered trademark) Sulfo-NHS-Biotin (13.5 Å, primary amine), EZ-Link (registered trademark) Sulfo-NHS-LC-Biotin (22.4 Å, primary amine), EZ-Link (registered trademark) Sulfo-NHS-LCLC-Biotin (30.5 Å, primary amine), EZ-Link (registered trademark) PFP-Biotin (9.6 Å, amine), EZ-Link (registered trademark) Maleimide-PEO₂-Biotin (29.1 Å, thiol group), EZ-Link (registered trademark) Biotin-PEO₂ Amine (20.4 Å, carboxyl group), EZ-Link (registered trademark) Biotin-PEO₃-LC Amine (22.9 Å, carboxyl group), EZ-Link (registered trademark) Biotin-Hydrazide (15.7 Å, aldehyde group), EZ-Link (registered trademark) Biotin-LC-Hydrazide (24.7 Å, aldehyde group), and EZ-Link (registered trademark) NHS-Iminobiotin (13.5 Å, primary amine).

Using such biotinylation reagent, the virus-binding substance such as a lectin can be linked to biotin by a known method.

For example, a biotinylation reagent containing an NHS ester is dissolved in an organic solvent such as DMSO (dimethyl sulfoxide) or a phosphate buffer having pH 7-9, and then the solution is added to the virus-binding substance such as a lectin, so that biotin can be linked to the virus-binding substance. Alternatively, in a case when a biotinylation reagent containing amino groups is used, biotin may be linked to the virus-binding substance by first changing carboxyl groups of the virus-binding substance such as a lectin to active ester using a carbodiimide such as 1-ethyl-3-(3-dimethylaminopropyl)carbodiimide hydrochloride (EDC) and then adding the biotinylation reagent dissolved in a buffer solution having a pH of about 5 to said virus-binding substance.

Instead, the biotinylated virus-binding substance such as a lectin may be purchased from, for example, J-Oil Mills Inc. as a biotin-labeled lectin. Alternatively, the biotinylated virus-binding substance may be produced by linking biotin to a desired virus-binding substance by using a biotin labeling kit (for example, though not limited to, EZ-Link (registered trademark) NHS-Lc-Biotin, a product of Pierce Inc. or Biotin Labeling Kit-NH2, a product of Dojindo Molecular Technologies, Inc.).

Alternatively, for example, a biotinylated virus-binding substance such as a lectin may be produced through fusion of a gene of a lectin to a DNA encoding a peptide comprising a biotinylated sequence, construction of a vector expressing this fused gene, and expression of a fusion protein having the biotinylated sequence in a host (Schwarz et al., J. Biol. Chem., (1988) 263: 9640-9645). Examples of the vector include, but not limited to, vectors containing a BioEase (trademark) tag, which is a product of Invitrogen Inc. Among such vectors, a pcDNA (trademark) 6 vector for mammalian cell expression, a pET104 vector for E. coli expression, and a pMT/BioEase vector for Drosophila expression; can be utilized.

In the present invention, any proteins capable of binding to biotin to form biotin/avidin bond, such as avidin, streptavidin, neutravidin, AVR protein (Biochem. J., (2002) 363: 609-617), bradavidin (J. Biol. Chem., (2005) 280: 13250-13255), rhizavidin (Biochem. J., (2007) 405: 397-405), tamavidin, and variants thereof, may preferably be used. In particular, tamavidin and its variants can be preferably used. Tamavidin is a biotin-binding protein that was found in basidiomycete Pleurotus cornucopiae, which is an edible mushroom (WO2002/072817; Takakura et al., FEBS J., (2009) 276: 1383-1397). An example of the variant of tamavidin is high affinity/low non-specific binding tamavidin (Japanese Patent Application No. 2008-208766, not yet published).

The term "tamavidin" in the present invention refers to tamavidin 1 (an amino acid sequence: SEQ ID NO: 11, a nucleic acid sequence encoding thereof: SEQ ID NO: 10), tamavidin 2 (an amino acid sequence: SEQ ID NO: 13, a nucleic acid sequence encoding thereof: SEQ ID NO: 12), or a variant thereof. Specifically, the tamavidin of the present invention may be typically a protein comprising the amino acid sequence of SEQ ID NO: 11 or SEQ ID NO: 13 or a protein encoded by a nucleic acid comprising the nucleotide sequence of SEQ ID NO: 10 or SEQ ID NO: 12. Alternatively, the tamavidin of the present invention may be a protein that is a variant of the protein comprising the amino acid sequence of SEQ ID NO: 11 or SEQ ID NO: 13 or the protein encoded by a nucleic acid comprising the nucleotide sequence of SEQ ID NO: 10 or SEQ ID NO: 12 and that has a biotin-binding activity similar to that of tamavidin 1 or 2 or a high affinity/low non-specific binding activity. Throughout the description, tamavidin 1, tamavidin 2, and a variant thereof may be referred to collectively as tamavidin.

The variant of tamavidin 1 or 2 may be a protein containing an amino acid sequence having deletion, substitution, insertion, and/or addition of one or more amino acids in the amino acid sequence of SEQ ID NO: 11 or 13 and having a biotin-binding activity similar to that of tamavidin 1 or 2. The substitution may be a conservative substitution where a specific amino acid residue is substituted by a residue having similar physiochemical characteristics. Nonlimiting examples of the conservative substitution include substitution between amino acid residues having aliphatic groups such as mutual substitution among Ile, Val, Leu, or Ala; and substitution between polar residues such as mutual substitution between Lys and Arg, Glu and Asp, or Gln and Asn.

The variant by deletion, substitution, insertion, and/or addition of amino acid(s) can be produced by, for example, induction of site-specific mutagenesis, which is a well-known technique, (for example, see Nucleic Acid Research, Vol. 10, No. 20, p.6487-6500, 1982, the entire content of which is incorporated in this description by reference), to a DNA encoding a wild-type protein. In this description, the term "one or more amino acids" refers to an amino acid or amino acids that allow deletion, substitution, insertion, and/or addition by the site-specific mutagenesis. In addition, the term "one or more amino acids" in this description may refer to one or several amino acids as appropriate.

Furthermore, the variant of tamavidin 1 or 2 may be a protein containing an amino acid sequence having a homology of at least 60%, preferably at least 65%, at least 70%, at least 75%, at least 80%, at least 85%, at least 90%, at least 95%, at least 96%, at least 97%, at least 98%, or at least 99%, and more preferably at least 99.3% with the amino acid sequence of SEQ ID NO: 11 or 13 and having a biotin-binding activity similar to that of tamavidin 1 or 2 or a high affinity/low non-specific binding activity.

The homology percentage between two amino acid sequences may be determined by visual inspection and mathematical computation. Alternatively, the identity percentage between two protein sequences may be determined based on the algorithm of Needleman, S. B. and Wunsch, C. D. (J. Mol. Biol., (1970) 48: 443-453) and comparison of sequence information using a GAP computer program, available from the University of Wisconsin Genetics Computer Group (UWGCG). Preferred default parameters of the GAP program include (1) scoring matrix, blosum 62, described in Henikoff, S. and Henikoff, J. G. (Proc. Natl. Acad. Sci. USA, (1992) 89: 10915-10919); (2) a gap weight of 12; (3) a gap length weight of 4; and (4) no penalty for end gap.

Other programs for sequence comparison that are used by those skilled in the art may also be used. The identity percentage can be determined by comparison of sequence information using, for example, the BLAST program described by Altschul, et al. (Nucl. Acids. Res., (1997) 25: 3389-3402). The program can be used from the website, National Center for Biotechnology Information (NCBI) or DNA Data Bank of Japan (DDBJ), on the Internet. Each conditions (parameters) for identity screening by the BLAST program is shown in the site in detail. Though the conditions may be modified partially, screening generally is performed using default values. Furthermore, the identity percentage between two amino acid sequences may be determined using a program such as the genetic information processing software GENETYX Ver. 7 (Genetyx Company) or FASTA algorithm. In such a case, default values may be used for the screening.

The identity percentage between two nucleic acid sequences can be determined by visual inspection and mathematical computation, or, more preferably, the sequence information is compared using a computer program. A typical and preferred computer program is the Wisconsin package of the genetic computer group (GCG; Wisconsin, Madison), version 10.0 program "GAP" (Devereux, et al., Nucl. Acids Res., (1984) 12: 387). Using this "GAP" program, in addition to comparison of two nucleic acid sequences, comparison of two amino acid sequences and comparison of a nucleic acid sequence and an amino acid sequence can be performed.

In the present invention, examples of preferable tamavidin include the following tamavidin modifications (Japanese Patent Application No. 2008-208766, not yet published):

A modified biotin-binding protein containing an amino acid sequence set forth in SEQ ID NO: 13, or, an amino acid sequence having one or several amino acid modifications in this sequence or an amino acid sequence having an identity of 80% or more with this sequence and showing a biotin-binding activity, wherein one or more residues selected from the following group:
1) an arginine residue at position 104 of SEQ ID NO: 13;
2) a lysine residue at position 141 of SEQ ID NO: 13;
3) a lysine residue at position 26 of SEQ ID NO: 13; and
4) a lysine residue at position 73 of SEQ ID NO: 13,
are substituted by an acidic or neutral amino acid residue.

More preferably, the modified biotin-binding protein is selected from the group consisting of:
a modified biotin-binding protein (R104E-K141E) where, in SEQ ID NO: 13, the arginine residue at position 104 is substituted by a glutamic acid residue and the lysine residue at position 141 is substituted by a glutamic acid residue;
a modified biotin-binding protein (D40N-R104E) where, in SEQ ID NO: 13, the aspartic acid residue at position 40 is substituted by an asparagine residue and the arginine residue at position 104 is substituted by a glutamic acid residue;
a modified biotin-binding protein (D40N-K141E) where, in SEQ ID NO: 13, the aspartic acid residue at position 40 is substituted by an asparagine residue and the lysine residue at position 141 is substituted by a glutamic acid residue; and
a modified biotin-binding protein (D40N-R104E-K141E) where, in SEQ ID NO: 13, the aspartic acid residue at position 40 is substituted by an asparagine residue, the arginine residue at position 104 is substituted by a glutamic acid residue, and the lysine residue at position 141 is substituted by a glutamic acid residue.

The biotin-binding protein and the carrier can be linked by using a method for coupling a protein and a carrier, which is known to those skilled in the art. For example, the protein and the carrier can be linked by modifying the carrier surface so as to expose carboxyl groups at the surface and coupling the carboxyl groups to amino groups of the protein in the presence of 1-ethyl-3-(3-dimethylaminopropyl)carbodiimide (EDC) serving as a cross-linking reagent. Alternatively, the carboxyl groups of the carrier surface and the amino groups of the protein can be bound by actively esterifying the carboxyl groups of the carrier surface by N-hydroxysuccinimide (NHS) and mixing the carrier with the protein in a buffer that does not contain primary amino groups and having a pH of 6.5 to 9.

Alternatively, amino groups of the carrier surface and amino groups of the protein can be bound using a cross-linking reagent, BS3 (bis[sulfosuccinimidyl] suberate) or DSS (disuccinimidyl suberate ); or amino groups of the carrier surface and thiol groups of the protein can be linked by using a cross-linking reagent, SPDP (N-succinimidyl 3-[2-pyridyldithio]propionate) or GMBS (N-(4-maleimidebutyryloxy)succinimide).

In a case when immobilizing the biotin-binding protein to a carrier, various commercially available carriers having various functional groups at the surfaces can be preferably utilized. Examples of microplates having functional groups on the surfaces include, but are not limited to, maleic anhydride plates such as Reacti-Bind (trademark) Maleic Anhydride Activated Polystyrene 96-Well Plates (Pierce Inc.), active amino group plates such as Immobilizer^{™}-Amino Modules/Plates (Nunc Inc.), and carboxyl group plates such as ELISA plate MS-8796F (96-well C-type/Flat bottom/Carbo) (Sumitomo Bakelite Co., Ltd.). Furthermore, examples of microbeads having a functional group on the surface include, but not limited to, highly cross-linked agarose beads such as Sepharose (trademark) (GE Healthcare Bio-Sciences Ltd.) and magnetic beads such as Dynabeads (trademark) (Dynal Inc.). The biotin-binding protein and the solid carrier can be linked in accordance with instructions attached to the carrier.

Alternatively, examples of the biotin-binding protein-immobilized carrier include, but not limited to, marketed products such as microplates, e.g., Reacti-Bind^{™} Streptavidin Coated Plates (Pierce Inc.) and Nunc Streptavidin Coated 96 Micro Well^{™} Plates (Nalge Nunc Inc.) and magnetic beads, e.g., Dynabeads M-280 Streptavidin (Dynal Inc) and MagnaBind^{™} Streptavidin Beads (Pierce Inc.).

In the case where the biotin and the biotin-binding protein are not used, the virus-binding substance and the carrier can also be indirectly bound by, for example, linking the virus-binding substance to a histidine tag, a FLAG^{™} tag, or glutathione-S-transferase (GST) and also linking the carrier to the corresponding Ni-NTA (nitrilotriacetic acid), anti-FLAG antibody, and glutathione, and then binding the virus-binding substance and the carrier.

### Standard virus

In the method of the present invention, the recovery rate of a virus to be quantitatively determined is not necessarily 100% and may vary subtly depending on samples and conditions. The recovery rate is therefore not exactly constant. This is satisfactory for the purpose of approximate determination of a number of virus on a single occasion. However, in order to quantitatively determine an amount of a virus for evaluating medium-and long-term fatigue, it is necessary to measure the amounts of the virus in body fluids collected at certain intervals of time and to continue with comparison of the amounts. Variation in the recovery rate therefore causes a problem which is a difficulty in the comparison of the amounts of the virus that is successively collected and quantitatively determined.

Through investigation, the inventors have successfully solved the problem by introduction to a system of a virus (hereinafter, referred to as "standard virus") that shows a recovery rate similar to that of a virus to be quantitatively determined and allows measurement of a number of the virus to be determined and a number of the standard virus independently under coexisting conditions. Specifically, a predetermined amount of the standard virus is added to an initial sample (for example, saliva), and the method of the present invention is performed. Then, the recovery rate is evaluated based on the amount of the standard virus added to the initial sample and the amount of the recovered standard virus. The amount of the target virus could be accurately determined constantly by multiplying the measured value of the target virus by the reciprocal of the recovery rate to correct the measured value of the amount of the virus.

Furthermore, the correction of the measured value of the target virus by adding the standard virus to the sample and evaluating the recovery rate is preferably conducted in one measurement system. Alternatively, it may be conducted by, for example, dividing a sample into two fractions, adding the standard virus to only one fraction for evaluating the recovery rate, measuring the target virus to be quantitatively determined in the other fraction, and correcting the measured value of the target virus based on the results obtained from these fractions.

In addition, when a large number of samples of a virus is measured by the method of the present invention, it is preferable to conduct the quantitative determination by introducing the standard virus to every sample. If the virus recovery rate is stable, however, the standard virus is not necessarily introduced to every sample. The introduction of the standard virus may be omitted in such a case. That is, while the stability of the recovery rate of a system is confirmed by introducing the standard virus to parts of the samples, the other samples may be subjected to quantitative determination without introducing the standard virus. The method of the present invention also includes such an embodiment.

It is necessary for the standard virus to have characteristics similar to those of the target virus to be quantitatively determined and to be able to be distinguished readily from the target virus. That is, in the method of quantitative determination of the present invention, it is necessary for the standard virus to bind to the virus-binding substance and be recovered using beads, similar to the target virus, and can readily be measured by, for example, PCR, LAMP, or ELISA, independently from the target virus.

The inventors have found that a mutated HHV-6 is a suitable standard virus satisfying the above-described requirements when HHV-6 is measured, and a mutated HHV-7 is suitable when HHV-7 is measured.

The mutated HHV-6 is preferably a recombinant virus derived from HHV-6 and having exogenous nucleotide sequences that are not present in the wild-type HHV-6 genome at positions corresponding to the U2 to U8 regions or at positions corresponding to the U24 and U25 regions of HHV-6. The mutated HHV-7 is preferably a recombinant virus derived from HHV-7 and having exogenous nucleotide sequences that are not present in the wild-type HHV-7 genome at positions corresponding to the U2 to U8 regions or the U24 and U25 regions of HHV-7.

When the virus is quantitatively determined by a system that detects nucleotide itself at high sensitivity, such as PCR or LAMP, the exogenous nucleotide sequence is not limited as long as it is a sequence that is not present in the genome of the wild-type HHV being a target to be quantitatively determined and in the human genome, and may not be a structural gene. In this case, a length of the exogenous nucleotide sequence is not particularly limited, but preferably ranges from 100 to 20,000 bases and more preferably from 200 to 10,000 bases. In such a system, when a structural gene is inserted as the exogenous nucleotide sequence, the gene is preferably a nucleotide sequence encoding a protein that does not affect a recovery rate of the HHV being the measuring target (for example, does not affect the HHV-6 sugar chain-lectin bond). Examples of the protein include, but are not limited to, fluorescent proteins, such as green fluorescent protein (GFP) and enhanced green fluorescent protein (EGFP); and antibiotic resistance proteins, such as proteins resistant to tetracycline, ampicillin, kanamycin, neomycin, hygromycin, or spectinomycin.

In addition, when a virus is quantitatively determined using an antibody against the virus, for example, by immunoassay such as ELISA, the mutated HHV is required to express an exogenous protein that is not present in the wild-type HHV. Such a protein is not particularly limited as long as it is encoded by an exogenous nucleotide sequence that is not present in the genome of the wild-type HHV to be quantitatively determined, but is preferably a sequence that is not present in the human genome. Furthermore, the exogenous protein is preferably, for example, the above-mentioned fluorescent protein or antibiotic resistance protein that does not affect the recovery rate of the HHV being the measuring target. Alternatively, an enzyme or the like that allows easy assay can preferably be used.

Preferred examples of such a mutated HHV-6 are recombinant viruses introduced with a gene encoding a protein for being measured independently from the wild-type HHV-6 (for example, see Japanese Patent No. 3923505). Preferred examples of such a mutated HHV-7 are recombinant viruses introduced with a gene encoding a protein for being measured independently from the wild-type HHV-7 (for example, see Japanese Patent Public Disclosure No. 2007-159586).

The amount of the standard virus added to a sample is not particularly limited as long as it allows quantitative determination of the standard virus, but, in the case of a mutated HHV-6, for example, the standard virus can be quantitatively determined when the amount is about 10 to 1,000,000 copies per mL, preferably about 10 to 100,000 copies per mL, more preferably about 50 to 50,000 copies per mL, and most preferably about 100 to 10,000 copies per mL.

In the quantitative determination using the standard virus, the standard virus, which has the above-mentioned characteristics, not only can be used as a standard of recovery or concentration of the target virus using a carrier, but also preferably can be used without modification as an internal standard in the subsequent quantitative determination by PCR or LAMP or a standard of the subsequent quantitative determination by ELISA.

### Contact of virus-binding substance with body fluid

The method of the present invention includes a step of bringing a body fluid into contact with the virus-binding substance. The conditions for contacting the body fluid and the virus-binding substance are not particularly limited as long as the virus in the sample binds to the virus-binding substance at a degree that allows quantitative determination of the virus. The factors of the contact conditions include incubation temperature and incubation time.

Furthermore, the body fluid may be brought into contact with the virus-binding substance directly or after addition of a desired buffer solution or the like to the body fluid. In a case where a desired buffer or any other additive is added to the body fluid, it is necessary for the amount of the virus to be calculated by taking into consideration the amount of the buffer added.

The lower limit of the incubation temperature may be 4°C or 10°C, while the upper limit may be selected from the group consisting of 50°C, 45°C, 40°C, 37°C, 30°C, 25°C, and 20°C. For example, preferred incubation temperature ranges from 10°C to 37°C, 10°C to 25°C, 10°C to 20°C, or 15°C.

The lower limit of the incubation time may be selected from the group consisting of 1 min, 3 min, 5 min, 10 min, 15 min, 20 min, and 30 min, while the upper limit may be selected from the group consisting of overnight, 10 h, 8 h, 5 h, 3 h, 2 h, 1.5 h, and 1 h. Examples of preferred incubation time ranges from 5 min to 2 h, from 10 min to 1.5 h, and from 30 min to 1 h.

In an embodiment where the virus-binding substance is indirectly immobilized to the carrier in the method of the present invention, the virus-binding substance and the carrier are brought into contact with and bound to each other through a molecule capable of binding to the carrier, before, while, or after the virus-binding substance comes into contact with the body fluid. The conditions for the contact of the virus-binding substance and the carrier are not limited particularly as long as the virus-binding substance can be sufficiently immobilized to the carrier through the molecule capable of binding to the carrier. The factors of the contact conditions include incubation temperature and incubation time. Conditions similar to those for the contact of the virus and the virus-binding substance described above can be selected as appropriate, but a most preferred incubation time ranges from 1 min to 2 h, from 3 min to 1 h, or from 5 to 30 min.

### Separation of carrier

The carrier that has captured the virus to be measured through the virus-binding substance is separated from the body fluid sample in a manner known to those skilled in the art according to the properties of the carrier.

For example, in the case of a particulate carrier such as a bead carrier, the carrier may be separated from the sample by centrifugation and subsequent removal of the supernatant. For a carrier that is in a magnetic bead form, the carrier may be separated from the sample by collecting the carrier using a magnet and removing the supernatant. For a carrier that is in a shape of a thin film or filter, the carrier may be separated from the sample by pulling up the carrier from the sample or may be separated from the sample after allowing the sample to pass through the carrier. For a carrier that is designed to capture the virus inside a well structure such as a microplate, the carrier may be separated from the sample merely by removing the sample from the well.

Furthermore, the step of washing the separated carrier may be conducted in order to remove substances non-specifically bound to the carrier that has been separated from the sample, according to need. The conditions of the washing solution and the temperature used in the washing step are not limited particularly as long as the conditions can maintain the binding between the virus and the virus-binding substance and/or the binding between the virus-binding substance and the carrier.

Preferably, the washing solution is buffered and is a buffer solution having a pH of 7 to 8, such as a buffer solution based on Tris/EDTA (TE), PBS, HEPES, or TBS. The pH of the washing solution may be 6 to 9, preferably 7 to 8.

The washing temperature is not limited particularly, but the lower limit of the washing temperature may be 4°C or 10°C while the upper limit may be selected from the group consisting of 50°C, 45°C, 40°C, 37°C, 30°C, 25°C, and 20°C. For example, the preferred washing temperature ranges from 10°C to 37°C, from 10°C to 25°C, 10°C to 20°C, or 15°C.

### Quantitative determination of concentrated virus solution

The carrier that has captured the virus to be measured is separated from the body fluid sample and/or is washed, and then a buffer solution is added to the carrier in an amount less than the initial volume of the body fluid. This procedure enhances the HHV concentration in the recovered sample so as to be higher than that in the body fluid. An amount less than the initial volume of the body fluid is not particularly limited, but may be 1/2, 1/10, 1/20, 1/40, 1/60, 1/80, or 1/100 of the initial volume.

The buffer solution added to the carrier that has captured the virus to be measured is selected appropriately in accordance with a method which is subsequently to be applied for determination of the virus.

After concentrating the HHV in the recovered sample to a level higher than the concentration in the body fluid as described above, the sample is subjected to quantitative determination of the number of the virus. In this description, the term "quantitative determination of the number of the virus" refers to quantitative determination of the amount of the virus present in a sample. Accordingly, the term "quantitative determination of the number of the virus" includes direct or indirect quantitative determination, for example, as the absolute number of the virus, the concentration of the virus, or the titer of the virus. The number of virus can be determined by any known method, for example, a method of quantitatively determining the amount of viral DNA or a method of quantitatively determining the number of antigen molecules derived from virus particles. Examples of the former method include methods based on PCR such as real-time PCR (Science, (1985), 230: 1350-1354) and quantitative determination of DNA employing, for example, LAMP method (Nucleic Acids Res., (2000), 28: E63). Examples of the latter method include quantitative determination of antigen proteins using, for example, sandwich ELISA. Furthermore, the quantitative determination may be performed as a titer of a virus by infecting desired cells with the virus.

Note that in the case of measuring an amount of the viral DNA, disruption of the virus coat protein is necessary. For the disruption, though not limited to, heat treatment, addition of a surfactant, proteinase K treatment, or a combination thereof can preferably be carried out in a suitable buffer solution.

The buffer solution is not particularly limited, and a pH for the reaction may range from 5.0 to 9.0, preferably from 6.0 to 8.0, and more preferably from 7.0 to 8.0. A buffer solution having a pH of 7 to 8 may be a buffer solution based on, for example, Tris/EDTA (TE), PBS, HEPES, or TBS. A temperature employed for heat treatment is not particularly limited, but may range preferably from 60°C to 100°C, more preferably from 70°C to 95°C, and most preferably from 80°C to 95°C. The treatment time ranges from 5 min to 1 h and preferably from 10 to 30 min. The type of the surfactant is not particularly limited as long as it does not affect subsequent quantitative determination of the virus. Examples thereof may include Nonidet P-40, Tween 20, and Triton X-100. A concentration of each surfactant may range from 0.01 to 1%, preferably from 0.05 to 0.5%, and more preferably from 0.05 to 0.25%. The heat treatment and the addition of a surfactant can be properly combined within the above-mentioned ranges.

### 2. Kit

The present invention also provides a kit for quantitatively determining the number of human herpesvirus collected from a body fluid based on the method of the present invention.

In one embodiment, the kit of the present invention includes at least a biotinylated virus-binding substance and a biotin-binding protein-immobilized carrier. Preferably, the kit of the present invention includes at least a biotinylated virus-binding substance, a biotin-binding protein-immobilized carrier, and a standard virus having a predetermined concentration. The virus-binding substance, the biotin-binding protein, the carrier, and the standard virus are as defined above.

In another embodiment, the kit of the present invention includes at least nanobeads to which the virus-binding substance is immobilized. Preferably, the kit of the present invention includes at least the nanobeads to which the virus-binding substance is immobilized and a standard virus having a predetermined concentration. The virus-binding substance, the nanobeads, and the standard virus are as defined above.

The kit of the present invention may further include a buffer solution for binding a virus and the virus-binding substance, a buffer solution for washing the separated virus-binding carrier, and/or a buffer solution for being subsequently added to the carrier, in the method of the present invention.

The kit of the present invention further includes a reagent and/or a buffer solution for quantitatively determining a virus. For example, when a virus is quantitatively determined based on quantitative determination of the amount of DNA, the reagent and/or the buffer solution are, for example, a DNA polymerase, a primer, a suitable probe according to need, and a suitable buffer solution. When a virus is quantitatively determined based on quantitative determination of the number of antigen molecules, the reagent and/or the buffer solution are, for example, an antibody against the antigen, a secondary antibody suitable for detection, and a suitable buffer solution.

In the kit of the present invention, each reagent may be contained in a proper container. In addition, the kit of the present invention may include a package for properly wrapping the reagents included in the kit.

Furthermore, the kit of the present invention may include suitable instructions for use. Nonlimiting examples of the instructions for use include media that can convey how to use the kit of the present invention to a user, such as description on the package, printed matter, an electronic memory medium (for example, magnetic disk, tape, cartridge, or chip), and an optical medium (for example, CD ROM). In addition, description of the address of an Internet site that provides instructions for use is included in the instructions for use.

### EXAMPLES

The present invention will be described based on examples in more detail, but those examples are not intended to limit the technical scope of the present invention. Those skilled in the art can modify or change the present invention readily based on the description of the present description, and such modifications and changes are included in the technical scope of the present invention.

### Example 1 Screening of lectin that can detect HHV

Enrichment of HHV-6 was investigated by allowing a biotinylated lectin to react with a cultured HHV-6 in solution and then to bind to tamavidin-immobilized magnetic beads.

### 1. Preparation of HHV-6 solution from cultured T cells

Cultured human cord blood-derived T cells were infected with recombinant HHV-6 expressing EGFP (Japanese Patent No. 3923505) to produce an EGFP-type HHV-6 solution.

### 2. Preparation of tamavidin magnetic beads

Three hundred microliters of magnetic beads having surfaces coated with carboxyl groups (Dynabeads M-270 Carboxylic Acid, Dynal Inc.) were washed with 300 µL of 0.01 N sodium hydroxide for 10 min and then with 300 µL of ultrapure water for 10 min three times. To the washed magnetic beads, 1-ethyl-3-(3-dimethylaminopropyl)carbodiimide hydrochloride (EDC) (Pierce Inc.) dissolved in cooled ultrapure water was added into a final concentration of 0.2 M, followed by shaking at room temperature for 30 min. Then, the magnetic beads were washed with 300 µL of cooled ultrapure water and then with 300 µL of 50 mM MES buffer solution (pH 5.0), and then mixed with 300 µL (180 µg) of 0.6 mg/mL tamavidin substituted by 50 mM MES buffer solution (pH 5.0). The mixture was shaken at room temperature for 30 min to link the tamavidin and the magnetic beads by covalent bonds. The magnetic beads were collected with a magnet, and the supernatant was removed. Then, unreacted carboxyl groups of the beads were eliminated by 300 µL of 50 mM tris buffer solution (pH 7.0), and the magnetic beads were blocked with 300 µL of a PBS buffer solution containing 0.5% BSA and 0.1% Tween 20. The magnetic beads were suspended in 300 µL of a PBS buffer solution to complete the preparation of magnetic beads. The biotin-binding activity of the tamavidin magnetic beads was 15 nmol per mL of the bead suspension.

### 3. Enrichment of recombinant HHV-6 solution

It was tried to raise the concentration of a virus by using the EGFP-type HHV-6 solution prepared in Section 1 described above and by using the infectious titer of the recombinant HHV-6 as an index. The biotinylated lectins used were the following 15 types: (Con A, DBA, LCA, PHA-E4, PNA, RCA120, UEA-I, WGA, ABA, DSA, Lotus, MAM, PHA-L4, SBA, and SSA) manufactured by J-Oil Mills Inc.

Before the screening of lectins, non-specific adsorption of the tamavidin causing background was confirmed.

First, 100 µL of the EGFP recombinant HHV-6 solution (concentration: 10⁴ copies/mL TE) and 500 µL of PBS were mixed and incubated at 15°C for 1 h (upside-down mixing). Then, the tamavidin magnetic beads prepared in Section 2 were added to the reaction solution, followed by incubation at 15°C for 1 h (upside-down mixing). Then, the Eppendorf tube containing the reaction solution was placed in a magnetic stand for Dynabeads, and the beads was washed with 200 µL of PBS containing 2 mM EDTA and 0.5% BSA twice. Then, the beads was suspended in 500 µL of a culture solution (RPMI1640 supplemented with 10% fetal bovine serum), and the whole suspension was mixed with 0.5 mL of an indicator cell (MT4 cell) suspension. It is believed that, if the EGFP-type HHV-6 is present, the indicator cells are infected with the EGFP-type HHV-6, the DNA of the HHV-6 enters the cells, and the EGFP gene incorporated in the HHV-6 is expressed within the indicator cells to generate GFP fluorescence.

Fig. 1 shows the experimental results confirming that almost no HHV-6 was non-specifically adsorbed to the tamavidin-immobilized beads.

Then, the possibility of increasing concentration of a virus was investigated using each lectin.

First, 100 µL of the EGFP recombinant HHV-6 solution (concentration: 10⁴ copies/mL TE), 500 µL of PBS, and 10 µg of a biotinylated lectin were mixed and incubated at 15°C for 1 h (upside-down mixing). Then, 10 µL of the tamavidin magnetic beads prepared in Section 2 were added to the reaction solution, followed by incubation at 15°C for 1 h (upside-down mixing). Then, the test tube containing the reaction solution was placed in a magnetic stand for Dynabeads, and the beads was washed with 200 µL of PBS containing 2 mM EDTA and 0.5% BSA twice. Then, the beads was suspended in 500 µL of a culture solution (RPMI1640 supplemented with 10% fetal bovine serum), and the whole suspension was mixed with 0.5 mL of indicator cell (MT4 cell) suspension. Expression of EGFP was observed. It is believed that, if the EGFP-type HHV-6 is concentrated, the indicator cells are infected with the EGFP-type HHV-6 bound to the magnetic beads through lectin-biotin-tamavidin binding, the DNA of the HHV-6 enters into the cells, and the EGFP gene incorporated in the HHV-6 is expressed within the indicator cells to generate GFP fluorescence.

Figs. 2 and 3 show the experimental results indicating that the expression of EGFP using SBA, SSA, DSA, or WGA was higher than that of the virus stock solution. These results suggest that HHV-6 can be efficiently concentrated by the use of these lectins.

### Example 2 Quantitative determination of HHV-6 in saliva without standard virus

The concentration of HHV-6 in saliva was quantitatively determined without using the standard virus but by utilizing the biotinylated lectin and the tamavidin magnetic beads.

### 1. Collection of saliva

Saliva was collected from a subject with Salivette (Salivette cotton, Sarstedt). The subject rinsed the oral cavity with distilled water twice immediately before the collection of saliva and put the inner cotton of the Salivette in the oral cavity to collect saliva for 2 min.

### 2. Quantitative determination of HHV-6 by conventional method

First, the concentration of HHV-6 in the saliva was quantitatively determined by a conventional method.

HHV-6 DNA in 400 µL of the saliva collected in Section 1 above was purified using BioRobot EZ1 (Qiagen Inc.) and EZ1 Virus Mini Kit v2.0 (Qiagen Inc.) in accordance with the protocol of EZ1 Virus Mini Handbook (Qiagen Inc.).

The resulting DNA was subjected to quantitative PCR. In the quantitative PCR, the HHV-6 U65/66 region was quantitatively determined by real-time PCR. The sequences used in the PCR were as follows:
Primer: 5'-GACAATCACATGCCTGGATAATG-3' (SEQ ID NO: 1);
Primer: 5'-TGTAAGCGTGTGGTAATGGACTAA-3' (SEQ ID NO: 2); and
TaqMan probe: FAM 5'-AGCAGCTGGCGAAAAGTGCTGTGC-3' TAMRA (SEQ ID NO: 3).
The real-time PCR was performed using the FastStart Universal Probe Master (Rox) (Roche Inc.) at a reaction temperature of 95°C for 10 min once, followed by 45 cycles of 95°C for 5 seconds and 60°C for 31 seconds.

This test was performed by an experimenter highly trained in virus experiment.

The determined HHV-6 DNA in 1 mL of saliva was 14616 copies/mL.

### 3. Quantitative determination of HHV-6 using biotinylated lectin and tamavidin

### magnetic beads

To 400 µL of saliva collected in Section 1 were mixed 44 µL of ten-fold concentration of PBS and 1 nmol of biotinylated SBA, followed by incubation at 15°C for 1 h (upside-down mixing). Then, 100 µL of the tamavidin magnetic beads prepared in Section 2 in Example 1 were added to the reaction solution, followed by further incubation at 15°C for 1 h (upside-down mixing). Then, the Eppendorf tube containing the reaction solution was placed in a magnetic stand for Dynabeads, and the beads was washed with 500 µL of PBS three times. Subsequently, 10 µL of TE was added to the beads, followed by treatment at 98°C for 10 min. Then, the supernatant was collected in the magnetic stand, and 5 µL of the supernatant was subjected to quantitative PCR. In the quantitative PCR, the HHV-6 U65/66 region was quantitatively determined by real-time PCR, as in Section 2.

The determined amount of HHV-6 in 5 µL of the eluate was 2934 copies. The calculated amount of HHV-6 in 10 µL ofTE is 5868 copies. Since this is the amount of HHV-6 present in 400 µL of the initial saliva, the converted amount per 1 mL is 14,670 copies/mL. This value corresponds with a value (14,616 copies/mL) measured by the conventional method in the above 2. In this case, it can be estimated that the recovery rate of the virus is approximately 100%.

As described above, HHV-6 can be quantitatively determined using the biotinylated SBA.

### Example 3. Quantitative determination of HHV-6 in saliva using standard virus

The concentration of HHV-6 in saliva was quantitatively determined using a biotinylated lectin, tamavidin magnetic beads, and a standard virus.

### 1. Method of collecting saliva

Saliva was collected from a subject with Salivette (Salivette cotton, Sarstedt). The subject rinsed the oral cavity with distilled water twice immediately before the collection of saliva, and placed the inner cotton of the Salivette into the oral cavity to collect saliva for 2 min.

### 2. Quantitative determination of HHV-6 by conventional method

The HHV-6 in the saliva was quantitatively determined as in Section 2 of Example 2.

It is to be noted that this test was carried out by an researcher with a high degree of training in virus experiments. The results are shown in Table 1.

### 3. Quantitative determination of HHV-6 using biotinylated lectin, tamavidin magnetic beads, and standard virus

### (1) Quantitative determination of HHV-6 using biotinylated WGA

To 400 µL of saliva collected in Section 1 was added an EGFP-type HHV-6 solution containing 1000 copies of EGFP-type HHV-6. Then, 44 µL of ten-fold concentration of PBS and 1 nmol of biotinylated WGA were mixed therewith, followed by incubation at 15°C for 30 min (upside-down mixing). Then, 100 µL of the tamavidin magnetic beads prepared in Example 1 were added to the reaction solution, followed by further incubation at 15°C for 30 min (upside-down mixing). Then, the Eppendorf tube containing the reaction solution was placed in a magnetic stand for Dynabeads, and the beads was washed with 500 µL of PBS three times. Then, 40 µL of proteinase K buffer (TE containing 1 mg/mL of proteinase K, 0.45% NP-40, and 0.45% Tween 20) was added thereto, followed by incubation at 56°C for 1 h to thereby destroy the virus particles for isolating the genomic DNA. Furthermore, the supernatant was collected in the magnetic stand. The supernatant was treated at 98°C for 10 min to inactivate the proteinase K, and 5 µL of the supernatant was subjected to quantitative PCR. In the quantitative PCR, the EGFP gene, which was present only in the EGFP recombinant virus, and the HHV-6 U5 gene, which was present in the wild-type HHV-6 but is deficient in the recombinant virus, were quantitatively determined by real-time PCR. The primers and Taqman probe used for quantitatively determining the EGFP gene were as follows:
Primer: 5'-CTGCTGCCCGACAACCA-3' (SEQ ID NO: 4);
Primer: 5'-TGTGATCGCGCTTCTCGTT-3' (SEQ ID NO: 5); and
TaqMan probe: FAM 5'-CTGAGCACCCAGTCCGCCCTG-3' TAMRA (SEQ ID NO: 6),
and the primers and Taqman probe used for quantitatively determining the HHV-6 U5 gene were as follows:
Primer: 5'-CGAAGAAAAGTAGCACAGGTCTCC-3' (SEQ ID NO: 7);
Primer: 5'-ACCGTGTCATAAATGCTGAGTTGG-3' (SEQ ID NO: 8); and
TaqMan probe: FAM 5'-AGGCACCCGTTCCGCCCCAGC-3' TAMRA (SEQ ID NO: 9).
The real-time PCR was performed using the FastStart Universal Probe Master (Rox) (Roche Inc.) at a reaction temperature of 95°C for 10 min once, followed by 45 cycles of 95°C for 5 seconds and 60°C for 31 seconds.

The recovery rate of the EGFP-type HHV-6 in each experiment was calculated from the ratio of the number of the EGFP-type HHV-6 quantitatively determined from the result of the real-time PCR of the EGFP gene to the initial number, namely, 1000 copies of the EGFP-type HHV-6. The value quantitatively determined by the method of the present invention was obtained by multiplying the number of the wild-type HHV-6 determined from the result of the real-time PCR of the HHV-6 U5 gene by the reciprocal of the recovery rate to correct the measured value.

The above-described experiment was carried out for three different saliva samples. Table 1 shows the results.

**[Table 1]**

| | Measured value of wild-type HHV-6 | Recovery rate of EGFP-type HHV-6 | Value of wild-type HHV-6 corrected by recovery rate | Measured value of wild-type HHV-6 by conventional method |
|---|---|---|---|---|
| Sample 1 | 220.3 | 0.58 | 379.8 | 397.3 |
| Sample 2 | 213.9 | 0.48 | 445.6 | 408.6 |
| Sample 3 | 2221.3 | 0.51 | 4355.4 | 4570.7 |

Table 1: Comparison of the results of quantitative determination of the number of HHV-6 using the biotinylated WGA and the tamavidin magnetic beads and the results of quantitative determination by a conventional method. (The units of numerical values other than the recovery rate are the number of HHV-6 genomic DNA copies in 1 mL of saliva.)

In every saliva sample, the value of the wild-type HHV-6 obtained by the method of the present invention and the value of the wild-type HHV-6 obtained by the conventional method were in close agreement with each other. This shows that quantitative determination of the number of HHV-6 in saliva can readily be performed by the method of the present invention.

### Example 4 Quantitative determination of HHV-7 in saliva without using standard virus

The concentration of HHV-7 in saliva was quantitatively determined by using a biotinylated lectin and the tamavidin magnetic beads, but without using the standard virus.

### 1. Collection of saliva

Saliva was collected from a subject with Salivette (Salivette cotton, Sarstedt). The subject rinsed the oral cavity with distilled water twice immediately before the collection of saliva, and placed the cotton of the Salivette into the oral cavity to collect saliva for 2 min.

### 2. Quantitative determination of HHV-7 by conventional method

First, the concentration of HHV-7 in the saliva was quantitatively determined by a conventional method.

HHV-7 DNA in 400 µL of the saliva collected in Section 1 above was purified using BioRobot EZ1 (Qiagen Inc.) and EZ1 Virus Mini Kit v2.0 (Qiagen Inc.) in accordance with the protocol of EZ1 Virus Mini Handbook (Qiagen Inc.).

The resulting DNA was subjected to quantitative PCR. In the quantitative PCR, the HHV-7 U37 region was quantitatively determined by real-time PCR. The sequences used in the PCR were as follows:
Primer: 5'- CGGAAGTCACTGGAGTAATGAC-3' (SEQ ID NO: 14);
Primer: 5'- CCAATCCTTCCGAAACCGAT-3' (SEQ ID NO: 15); and
TaqMan probe: FAM 5'-CCTCGCAGATTGCTTGTTGGCCATG-3' TAMRA (SEQ ID NO: 16).
The real-time PCR was performed using FastStart Universal Probe Master (Rox) (Roche Inc.) at a reaction temperature of 95°C for 10 min once, followed by 45 cycles of 95°C for 5 seconds and 60°C for 31 seconds.

This test was performed by an experimenter highly trained in virus experiment.

The determined HHV-7 DNA in 1 mL of saliva was 221,774 copies/mL.

### 3. Quantitative determination of HHV-7 by using biotinylated WGA and tamavidin magnetic beads

To 250 µL of saliva collected in Section 1 were mixed 250 µL of two-fold concentration of PBS and 20 µL of biotinylated WGA (J-Oil Mills Inc.), followed by incubation at 15°C for 1 h (upside-down mixing). Then, 50 µL of tamavidin magnetic beads prepared as in Section 2 in Example 1 (provided that Dynabeads MyOne was used as the magnetic beads) were added to the reaction solution, followed by further incubation at 15°C for 1 h (upside-down mixing). Then, the Eppendorf tube containing the reaction solution was placed in a magnetic stand for Dynabeads, and the beads was washed with 500 µL of PBS three times. Subsequently, 40 µL of TE containing 0.09% of Tween 20 was added to the solution, followed by treatment at 95°C for 15 min. Then, the supernatant was collected on the magnetic stand, and 5 µL of the supernatant was subjected to quantitative PCR. In the quantitative PCR, the HHV-7 U37 region was quantitatively determined by real-time PCR, as in Section 2.

The amount of measured HHV-7 in 1 mL of the saliva was measured to be 223,934 copies. This value well agreed with the value (221,774 copies/mL) measured by the conventional method in the above 2. In this case, it can be estimated that the recovery rate of the virus is approximately 99%.

As described above, HHV-7 can be quantitatively determined using the biotinylated WGA.

### INDUSTRIAL APPLICABILITY

The present invention provides a novel quantitative determination method that can measure the number of HHV in a body fluid more simply, accurately, and efficiently, and provides a kit for performing the method. The method of the present invention can undergo continuous evaluation of the number of HHV in body fluids and, therefore, can be applied to quantitative evaluation of the accumulation of fatigue.

## Claims

1. A method of quantitatively determining a number of human herpesvirus collected from a body fluid, the method comprising steps of:
(1) adding a standard virus to the collected body fluid, a concentration of the standard virus being determined in advance;
(2) bringing the solution prepared in step (1) into contact with a virus-binding substance, wherein (i) the virus-binding substance is linked to a molecule capable of binding to a carrier and is immobilized to the carrier through the molecule and thereby makes the virus particles bound to the virus-binding substance bind indirectly to the carrier, or (ii) the virus-binding substance is directly immobilized to a carrier and thereby makes the virus particles bound to the virus-binding substance bind to the carrier;
(3) separating the carrier from the solution prepared in step (1);
(4) quantitatively determining the number of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

2. A method of quantitatively determining the number of human herpesvirus collected from a body fluid, the method comprising the steps of:
(1) adding a standard virus to the collected body fluid, the concentration of the standard virus being determined in advance;
(2) bringing the solution prepared in step (1) into contact with a lectin, wherein (i) the lectin is linked to a molecule capable of binding to a carrier and is immobilized to the carrier through the molecule and thereby makes the virus particles bound to the lectin bind indirectly to the carrier, or (ii) the lectin is directly immobilized to a carrier and thereby makes the virus particles bound to the lectin bind to the carrier;
(3) separating the carrier from the solution prepared in step (1);
(4) quantitatively determining the numbers of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

3. A method of quantitatively determining the number of human herpesvirus collected from saliva, the method comprising the steps of:
(1) adding a standard virus to the collected saliva, the concentration of the standard virus being determined in advance;
(2) mixing the solution prepared in step (1) with a biotinylated lectin for bringing virus particles into contact with the biotinylated lectin, and then adding biotin-binding protein-immobilized beads thereto to make the virus particles bound to the biotinylated lectin bind to the beads;
(3) separating the beads from the solution prepared in step (1);
(4) quantitatively determining the numbers of virus recovered from the separated beads; and
(5) evaluating the recovery rate by comparing the number of the collected standard virus with the number of the standard virus added in step (1), and determining the number of the human herpesvirus collected from the saliva based on the number of the human herpesvirus determined in step (4) and the recovery rate.

4. The method according to any one of Claims 1 to 3, wherein the human herpesvirus is human herpesvirus 6 (HHV-6) or human herpesvirus 7 (HHV-7).

5. The method according to any one of Claims 1 to 3, wherein the standard virus is a recombinant virus derived from HHV-6 or HHV-7.

6. The method according to Claim 2 or 3, wherein the lectin is a lectin that binds to a sugar chain containing at least one ofN-acetylgalactosamine (GalNAc), α2,6-linked sialic acid (Siaα2,6), and N-acetylglucosamine (GlcNAc).

7. The method according to Claim 6, wherein the lectin is selected from the group consisting of SBA (derived from soybean), SSA (derived from *Sambucus sieboldiana*), DSA (derived from *Datura stramonium*), and WGA (derived from wheat germ).

8. The method according to any one of Claims 1 to 3, wherein the quantitative determination of the number of virus in step (4) is performed by a procedure selected from the group consisting of PCR, LAMP, and ELISA.

9. A method of quantitatively determining the number of human herpesvirus 6 (HHV-6) or human herpesvirus 7 (HHV-7) collected from saliva, the method comprising the steps of:
(1) adding a standard virus having a predetermined concentration to the collected saliva, such that the concentration of the standard virus is 10 to 100000 genome copies/mL, wherein the standard virus is a recombinant virus derived from HHV-6 or HHV-7;
(2) mixing the solution prepared in step (1) with a biotinylated lectin for bringing virus particles into contact with the biotinylated lectin, and then adding biotin-binding protein-immobilized beads thereto to make the virus particles bound to the biotinylated lectin bind to the beads, wherein the lectin is selected from the group consisting of SBA (derived from soybean), SSA (derived from *Sambucus sieboldiana*), DSA (derived from *Datura stramonium*), and WGA (derived from wheat germ);
(3) separating the beads from the solution prepared in step (1);
(4) quantitatively determining the number of virus recovered from the separated beads by a procedure selected from the group consisting of PCR, LAMP, and ELISA; and
(5) evaluating the recovery rate by comparing the number of the collected standard virus with the number of the standard virus added in step (1), and determining the number of the human herpesvirus collected from the saliva based on the number of the human herpesvirus determined in step (4) and the recovery rate.

10. A method of quantitatively determining the number of human herpesvirus collected from a body fluid, the method comprising the steps of:
(1) adding a standard virus to the collected body fluid, the concentration of the standard virus being determined in advance;
(2) bringing the solution prepared in step (1) into contact with a virus-binding substance that is directly immobilized to nanobeads having a diameter of 10 to 100 nm to make virus particles bind to the nanobeads;
(3) separating the nanobeads from the solution prepared in step (1);
(4) quantitatively determining the number of the virus recovered from the separated carrier; and
(5) evaluating the recovery rate by comparing the number of the collected standard virus with the number of the standard virus added in step (1), and determining the number of the human herpesvirus collected from the body fluid based on the number of the human herpesvirus determined in step (4) and the recovery rate.

11. A kit for quantitatively determining the number of human herpesvirus collected from a body fluid, the kit comprising:
a biotinylated lectin; and
biotin-binding protein-immobilized beads.

12. A kit for quantitatively determining the number of human herpesvirus collected from a body fluid, the kit comprising:
a biotinylated lectin;
biotin-binding protein-immobilized beads; and
a standard virus, the concentration of the standard virus being determined in advance.

13. The kit according to Claim 11 or 12, wherein the lectin is a lectin that binds to a sugar chain containing at least one ofN-acetylgalactosamine (GalNAc), α2,6-linked sialic acid (Siaα2,6), and N-acetylglucosamine (GlcNAc).

14. The kit according to Claim 13, wherein the lectin is selected from the group consisting of SBA (derived from soybean), SSA (derived from *Sambucus sieboldiana*), DSA (derived from *Datura stramonium*), and WGA (derived from wheat germ); and the standard virus is a recombinant virus derived from HHV-6 or HHV-7.

15. A method of quantitatively determining the number of human herpesvirus collected from a body fluid, the method comprising the steps of:
(1) bringing the collected body fluid into contact with a virus-binding substance, wherein (i) the virus-binding substance is linked to a molecule capable of binding to a carrier and makes the virus bound to the virus-binding substance indirectly bind to the carrier by that the virus-binding substance is immobilized to the carrier through the molecule, or (ii) the virus-binding substance is directly immobilized to a carrier and thereby makes the virus bound to the virus-binding substance bind to the carrier;
(2) separating the carrier from the solution prepared in step (1); and
(3) quantitatively determining the amount of the virus recovered from the separated carrier.

16. A method of quantitatively determining the number of human herpesvirus collected from saliva, the method comprising the steps of:
(1) mixing the collected saliva with a biotinylated lectin for bringing a virus into contact with the biotinylated lectin, and then adding biotin-binding protein-immobilized beads thereto to make the virus bound to the biotinylated lectin bind to the beads;
(2) separating the beads from the solution prepared in step (1); and
(3) quantitatively determining the number of the virus recovered from the separated beads.
